# EUROPÄISCHE PATENTANMELDUNG

(11) **EP 1 533 738 A2**
(43) Veröffentlichungstag der Anmeldung: **25.05.2005**
(21) Anmeldenummer: 04020073.5
(22) Anmeldetag: 24.08.2004
(51) Int. Cl.: G06F 19/00

(54) **Interaktives System zur Kontrolle und Protokollierung der Pflege und/oder Medikation mindestens zweier Personen**

(30) Priorität: 29.08.2003 DE 10339893
(71) Anmelder: Tulafor Anstalt, 9490 Vaduz (LI)
(72) Erfinder: Piwonka, Peter, 4011 Basel (CH)
(74) Vertreter: Körber, Martin, Dipl.-Phys.

(57) **Zusammenfassung**

Ein interaktives System zur Kontrolle und Protokollierung der Pflege und/oder Medikation mindestens zweier Personen enthält eine Datenbank (2), welche Information bezüglich der Art sowie des Zeitpunkts von an den Personen durchzuführenden Pflege- und/oder Medikationsmaßnahmen beinhaltet, eine Kontrollund Protokollierungseinheit (1), welche auf Basis der in der Datenbank (2) enthaltenen Information sowie unter Berücksichtigung von Eingaben des Pflegepersonals einen dem aktuellen Zeitpunkt entsprechenden Pflegestatus für die mindestens zwei Personen ermittelt, sowie eine Anzeigeeinheit (3), auf welcher der von der Kontroll- und Protokollierungseinheit (1) ermittelte Pflegestatus dargestellt ist.. Hierdurch wird die Möglichkeit geschaffen, auf einfache Weise die Pflege einer Vielzahl von Personen zu verwalten bzw. zu überwachen und dem Pflegepersonal die Möglichkeit zu geben, schnell festzustellen, welche Maßnahmen noch durchgeführt werden müssen.

## Beschreibung

Die vorliegende Erfindung betrifft ein System zur Kontrolle und Protokollierung der Pflege sowie der Medikation mindestens zweier Personen.

Im Hintergrund der vorliegenden Erfindung steht die Pflege von pflegebedürftigen Personen beispielsweise in Krankenhäusern oder in Alters- bzw. Pflegeheimen. Um diesen Personen eine optimale Pflege zukommen zu lassen, ist es erforderlich, zu festgelegten Zeitpunkten bestimmte Pflegemaßnahmen durchzuführen oder Medikamente zu verabreichen. Die für die verschiedenen Personen erforderlichen Pflegemaßnahmen können dabei sehr unterschiedlicher Natur sein und hängen unter anderem auch von den Krankheiten oder sonstigen Bedürfnissen der zu pflegenden Personen ab.

Aufgrund der unterschiedlichen Pflegeanforderungen verschiedener Personen ist es erforderlich, die durchzuführenden Maßnahmen genauestens zu planen und möglichst unmittelbar zu dem dafür vorgesehenen Zeitpunkt durchzuführen. Gleiches gilt auch für die Verabreichung von Medikamenten, wobei hier selbstverständlich besonders Wert auf eine zeitgenaue Verabreichung gelegt werden sollte. Die geplanten Maßnahmen sollten daher von dem Pflegepersonal möglichst einfach und übersichtlich eingesehen werden können, um den Arbeitsablauf optimal darauf abstimmen zu können.

Darüber hinaus wäre es wünschenswert, die durchgeführten Maßnahmen protokollieren zu können, um einerseits den behördlichen Auflagen genügen zu können und andererseits unmittelbar feststellen zu können, welche geplanten Pflegemaßnahmen bereits durchgeführt wurden bzw. welche Arbeiten noch durchzuführen sind.

Der vorliegenden Erfindung liegt dementsprechend die Aufgabe zugrunde, ein System anzugeben, welches die oben genannten Anforderungen erfüllt und somit eine optimale Kontrolle und Protokollierung der Pflege mehrerer Personen ermöglicht.

Die Aufgabe wird durch ein interaktives System gemäß Anspruch 1 gelöst. Dieses besteht im Wesentlichen aus drei Elementen, nämlich
- einer Datenbank, welche Informationen bezüglich der Art sowie des Zeitpunkts von an den zu pflegenden Personen durchzuführenden Pflege - und/oder Medikationsmaßnahmen beinhaltet,
- einer Kontroll- und Protokollierungseinheit, welche auf Basis der in der Datenbank enthaltenen Informationen sowie unter Berücksichtigung von Eingaben des Pflegepersonals einen dem aktuellen Zeitpunkt entsprechenden Pflegestatus für die zu pflegenden Personen ermittelt sowie
- einer Anzeigeeinheit, auf welcher der von der Kontroll- und Protokollierungseinheit ermittelte Pflegestatus dargestellt ist.

Wesentlicher Gedanke der vorliegenden Erfindung ist, dass die Kontroll- und Protokollierungseinheit zum einen über die Datenbank Informationen enthält, welche Art von Pflege- und/oder Medikationsmaßnahmen zu welchem Zeitpunkt vorgesehen sind, sowie zum anderen über Eingaben des Pflegepersonals informiert wird, welche Maßnahmen bereits zu welchen Zeitpunkten durchgeführt wurden. Anhand eines Vergleichs der geplanten Maßnahmen mit den bereits durchgeführten Maßnahmen ermittelt dann die Kontroll- und Protokollierungseinheit für jede zu pflegende Person einen Status, der angibt, ob die Pflege der Person den geplanten Maßnahmen entspricht oder ob noch Maßnahmen durchzuführen sind. Hierbei berücksichtigt die Kontrollund Protokollierungseinheit die zeitliche Entwicklung, so dass zu jedem Zeitpunkt der aktuell gültige Pflegestatus ermittelt wird. Mit Hilfe der Anzeigeeinheit wird dieser dann übersichtlich dargestellt, so dass für das Pflegepersonal unmittelbar ersichtlich ist, hinsichtlich welcher zu pflegenden Personen noch Handlungen durchzuführen sind. Das versehentliche Unterlassen einer Pflegemaßnahme oder der Verabreichung eines Medikaments wird hierdurch unterbunden, so dass ein hoher Pflegestandard gewährleistet ist.

Weiterbildungen der Erfindung sind Gegenstand der Unteransprüche.

Der von der Kontroll- und Protokollierungseinheit ermittelte Pflegestatus wird aus Gründen der Übersichtlichkeit auf der Anzeigeeinheit vorzugsweise symbolisch dargestellt und umfasst insbesondere eine farbliche Kodierung. Beispielsweise kann der von der Kontroll- und Protokollierungseinheit ermittelte Pflegestatus in drei verschiedene Kategorien eingeteilt werden, wobei die erste Kategorie einem optimalen Status entspricht, bei dem alle geplanten Maßnahmen erledigt wurden, eine zweite Kategorie einem Status entspricht, in dem noch nicht alle geplanten Maßnahmen durchgeführt wurden, die Verzögerung allerdings aufgrund bestimmter Voraussetzungen entschuldigt ist, und wobei gemäß einem dritten Pflegestatus bestimmte geplante Maßnahmen unentschuldigter Weise noch nicht durchgeführt wurden. Diese drei Kategorien können beispielsweise durch die Farben Grün, Gelb bzw. Braun und Rot kodiert werden, wobei vorzugsweise die Namen der zu pflegenden Personen auf der Anzeigeeinheit mit den dem zugeordneten Pflegestatus entsprechenden Farben hinterlegt sind. Das Pflegepersonal kann somit durch einen einfachen Blick auf die Anzeigeeinheit feststellen, bei welchen Personen noch Maßnahmen, deren verzögerte Durchführung nicht entschuldigt ist, vorzunehmen sind.

Gemäss einer einfachen Version des erfindungsgemäßen Kontrollsystems berücksichtigt die Kontroll- und Protokollierungseinheit ausschließlich die in der Datenbank enthaltenen Informationen sowie die Eingaben des Pflegepersonals, mit denen bestätigt bzw. nicht bestätigt wurde, welche der geplanten Pflegemaßnahmen durchgeführt wurden. Bei dieser Variante werden auch die in der Datenbank enthaltenen Informationen, also der Zeitplan für die bei den einzelnen Personen durchzuführenden Pflegeschritte ausschließlich durch das Pflegepersonal bzw. durch Ärzte vorgegeben, welche den Pflegeplan für die zu versorgenden Personen erstellen.

Gemäss einer Weiterentwicklung des Systems berücksichtigt die Kontroll- und Protokollierungseinheit allerdings zusätzlich noch externe Parameter, welche durch Überwachungsvorrichtungen übermittelt werden, die wiederum eine oder mehrere Körperfunktionen der zu pflegenden Personen überwachen. Bei den überwachten Körperfunktionen kann es sich beispielsweise um die Körpertemperatur, den Puls oder den Blutdruck der zu pflegenden Personen handeln. Die von der bzw. den Überwachungsvorrichtungen erfassten Daten werden an die Kontroll- und Protokollierungseinheit übermittelt, welche diese Daten berücksichtigt und den Pflegestatus in Abhängigkeit von den erfassten Daten ermittelt. Hierbei kann die Kontroll- und Protokollierungseinheit sogar den Pflegeplan für die überwachte Person überarbeiten oder ergänzen, wenn dies aufgrund der erfassten Messwerte erforderlich sein sollte. Beispielsweise könnte die Kontroll- und Protokollierungseinheit die Verabreichung eines Medikaments von einem späteren Zeitpunkt zu einem früheren Zeitpunkt verlegen, falls dies aufgrund eines festgestellten erhöhten Blutdrucks über einen längeren Zeitpunkt hinweg erforderlich ist.

Das vorliegende System ermöglicht somit eine optimierte Planung der durchzuführenden Pflegemaßnahmen. Gleichzeitig ist für das Pflegepersonal unmittelbar ersichtlich, bei welchen Personen noch Maßnahmen durchgeführt werden müssen, so dass die Durchführung der geplanten Maßnahmen zu den beabsichtigten Zeitpunkten weitestgehend sichergestellt ist. Ein versehentliches Vergessen einer Maßnahme ist nahezu ausgeschlossen.

Ein weiterer Vorteil des Systems besteht darin, dass die Durchführung der geplanten Pflegemaßnahmen automatisch protokolliert und - sofern gewünscht - ausgedruckt werden kann. Hierdurch kann zweifelsfrei nachgewiesen werden, von welcher Pflegekraft welche Maßnahme an welchem Patienten mit welchen Mitteln vorgenommen wurde bzw. welche Maßnahmen unterlassen wurden. Durch die Protokollierung der Tätigkeiten, die möglicherweise demnächst sogar gesetzlich verankert werden wird, kann die Überwachung der Qualität der Pflege deutlich verbessert werden. Darüber hinaus trägt die Protokollierungsfunktion auch dazu bei, den Aufwand und die für jeden Patienten angefallenen Pflegekosten zu erfassen. Diese Protokollierungsfunktion kann auch insbesondere bei der Verabreichung von Medikamenten zum Einsatz kommen, wobei hier als besondere Sicherungsfunktion vorgesehen sein kann, daß das verabreichte Medikament zunächst - beispielsweise mit Hilfe eines Scanners - eindeutig identifiziert werden muß, bevor es verabreicht werden darf. Hierdurch wird sichergestellt, daß nicht fälschlicherweise ein falsches Medikament verabreicht wird.

Eine andere Weiterbildung der Erfindung betrifft Maßnahmen, welche die unmittelbare Durchführung geplanter Pflegetätigkeiten unterstützen. So kann das erfindungsgemäße System zu einer Benutzeroberfläche erweitert werden, welche Informationen über die durchzuführenden Tätigkeiten zur Verfügung stellt. Wird z.B. eine Pflegetätigkeit angewählt, so erscheint auf der Anzeigeeinheit eine neue Seite oder ein weiteres Fenster mit genauen Anweisungen hinsichtlich der durchzuführenden Schritte in Wort und/oder Bild. Die dargestellten Informationen können dabei sowohl die Handlungen des Pflegepersonals als auch das zu verwendende Material betreffen.

Das auf diese Weise weitergebildete System unterstützt somit durch das Zurverfügungstellen von exakten Anweisungen die Tätigkeit des Pflegepersonals. Die ist deshalb von Vorteil, da in Zukunft die Pflege mehr und mehr auch durch unausgebildete Arbeitskräfte erfolgen wird, für die detaillierte Angaben über die durchzuführenden Maßnahmen unerläßlich sind. Im Gegensatz dazu wurden in der Vergangenheit und werden derzeit noch überwiegend ausgebildete Pflegekräfte eingesetzt.

Ein weiterer Vorteil der Darstellung der durchzuführenden Maßnahmen besteht auch darin, dass die zur Verfügung gestellten Information auf einfache und schnelle Weise überarbeitet oder ergänzt werden können. Hierdurch wird die sofortige Umsetzung neuer Pflegestandards ermöglicht und neue Materialien können ohne Verzögerung in den Arbeitsablauf eingebunden werden. Das bislang übliche Verteilen umfangreicher schriftlicher Informationen und langwierige Studium der Unterlagen wird hierdurch nahezu überflüssig.

Nachfolgend soll die Erfindung anhand der beiliegenden Zeichnung näher erläutert werden. Es zeigen:
- Fig. 1: ein Blockschaltbild, welches schematisch die einzelnen Elemente des erfindungsgemäßen Systems zum Überwachen und Protokollieren von Pflege- und/oder Medikationsmaßnahmen darstellt;
- Fig. 2: ein Beispiel einer auf der Anzeigeeinheit des erfindungsgemäßen Systems gezeigten Übersichtsdarstellung des ermittelten Pflegestatus für eine Vielzahl von Personen;
- Fig. 2a: eine erweiterte Darstellung eines Patientenbetts in der Übersichtsdarstellung von Fig. 2
- Fig. 3: eine Darstellung des für eine Person erstellten Pflegeplans;
- Fig. 4: eine von dem System zur Verfügung gestellte Benutzeroberfläche zur Protokollierung der bei einer Pflegemaßnahme durchzuführenden Handlungen;
- Fig. 5: eine erweiterte Medikations-Anzeige zur Planung der Verabreichung von Medikamenten;
- Fig. 6: ein Bestätigungsfenster zur expliziten Bestätigung von Änderungen der Medikation;
- Fig. 7: eine erweiterte Patientenzustandsanzeige zur Darstellung aller wesentlichen Informationen hinsichtlich des aktuellen Ist-Zustands eines Patienten; und
- Fig. 8: ein weiteres Ausführungsbeispiel eines Pflegeplans für eine bestimmte Person.

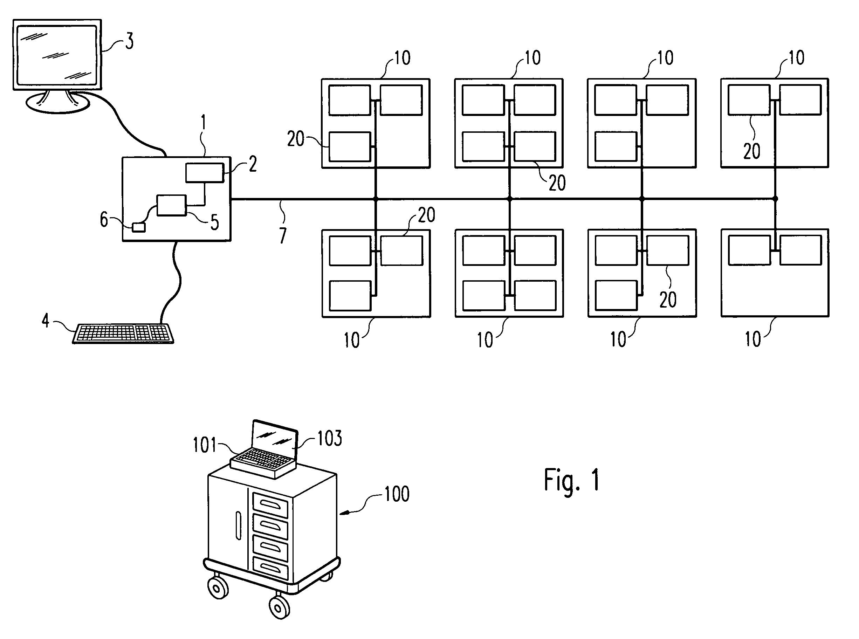

Fig. 1 zeigt zunächst schematisch die verschiedenen Bestandteile des erfindungsgemäßen Systems. Dieses besteht im Wesentlichen aus der Kontroll- und Protokollierungseinheit 1, die beispielsweise durch einen Computer gebildet wird und welche die Datenbank 2 beinhaltet, sowie aus der Anzeigeeinheit 3, auf welcher die von der Kontroll- und Protokollierungseinheit 1 erstellten Informationen dargestellt werden.

Die in der Kontroll- und Protokollierungseinheit 1 enthaltene Datenbank 2 beinhaltet - wie bereits erwähnt - Informationen bezüglich mehrerer Maßnahmen oder Handlungen, die an zu pflegenden Personen durchgeführt werden müssen. Bei diesen Maßnahmen kann es sich einerseits um Pflegemaßnahmen, wie beispielsweise das Wechseln eines Verbandes bzw. eines Katheters oder der Umlagerung der Person handeln. Andererseits enthält die Datenbank 2 allerdings auch Informationen hinsichtlich der Art, Menge bzw. Dosis sowie des Zeitpunkts zu verabreichender Medikamente. Für jede zu pflegende Person wird hierdurch ein Pflege- und Medikationsplan erstellt, der im Idealfall zeitgenau und vollständig von dem Pflegepersonal umgesetzt werden sollte.

Die für die einzelnen Personen geplanten Pflege- und Medikationsmaßnahmen werden dabei vorwiegend von dem Pflegepersonal oder den Ärzten erstellt und unmittelbar in das System eingegeben. Hierzu könnte beispielsweise eine Tastatur 4 verwendet werden, die an den die Kontroll- und Protokollierungseinheit 1 bildenden PC bzw. an die Anzeigeeinheit 3 angeschlossen ist. Vorzugsweise wird für die Anzeigeeinheit 3 allerdings ein sogenannter Touch-Screen verwendet, der die unmittelbare Eingabe von Informationen durch eine Berührung der Bildschirmoberfläche ermöglicht. Hierdurch wird ein besonders einfach zu bedienendes System gebildet.

Das erfindungsgemäße System zur Kontrolle- und Protokollierung von Pflege- und Medikationsmaßnahmen ist vorzugsweise in dem Stationszimmer einer Kranken- oder Pflegestation eines Krankenhauses oder eines Pflegeheims installiert. Die von der Kontroll- und Protokollierungseinheit 1 ermittelten und auf der Anzeigeeinheit 3 dargestellten Informationen beziehen sich dabei auf den Pflegestatus mehrerer Personen, die sich in den verschiedenen Zimmern der Kranken- oder Pflegestation befinden. Fig. 1 zeigt schematisch eine Station mit acht Krankenzimmern 10, in denen sich jeweils mehrere Betten 20 befinden. Sobald ein Bett 20 von einer Person belegt ist, erstellt die Kontroll- und Protokollierungseinheit 1 automatisch einen dazugehörigen Pflegestatus, der auf der Anzeigeeinheit 3 dargestellt ist.

Alternativ zu der in Fig. 1 dargestellten Ausführungsform kann sich die Überwachungs- und Protokollierungseinheit 1 auch an einem zentralen Ort des Krankenhauses befinden, wobei in diesem Fall lediglich die Anzeigeeinheit 3 in dem Stationszimmer angeordnet ist. Ferner können die Überwachungs- und Protokollierungseinheiten 1 verschiedener Stationen auch untereinander vernetzt sein, so dass auf die Ergebnisse und Daten anderer Stationen zugegriffen werden kann, was beispielsweise dann von Vorteil ist, wenn einige wenige Personen mehrere Stationen überwachen müssen.

Eine weitere Möglichkeit, die ebenfalls in Fig. 1 dargestellt ist, besteht darin, einen Computer 101 mit der darin befindlichen Überwachungs- und Protokollierungseinheit auf einem Pflegewagen 100 anzuordnen. Das Pflegepersonal, das den Wegen 100 mit sich führt, kann dabei jederzeit auf die Informationen und Daten des Systems zurückgreifen. Diese mobile Anordnung des erfindungsgemäßen Systems bietet sich insbesondere auch dann an, wenn vor Ort, also in den Patientenzimmern auf die Daten und Informationen des Systems zugegriffen werden muß und wenn die einzelnen Tätigkeiten einer Pflegemaßnahme von dem Personal bestätigt werden müssen. Dies wird weiter unten noch ausführlicher erläutert.

Der auf dem Pflegewagen 100 angeordnete Computer 101 könnte dabei auch eine drahtlose Kommunikationsverbindung zu dem fest installierten Gerät 1 in dem Stationszimmer aufbauen, um beispielsweise zentrale Informationen oder Patientendaten regelmäßig aktualisieren zu können. Der Bildschirm dieses Computers 101 ist wiederum vorzugsweise als Touch-Screen 103 ausgebildet.

Bei einer einfachen Variante des erfindungsgemäßen Systems berücksichtigt die Protokoll- und Protokollierungseinheit 1 ausschließlich manuell - entweder über die Tastatur 4 oder über die als Touch-Screen ausgebildete Anzeigeeinheit 3 - eingegebene Informationen. Dabei werden zum einen - wie bereits erwähnt - für jeden Patienten bestimmte Daten eingegeben, welche die Art und den Zeitpunkt von durchzuführenden Pflege- und Medikationsmaßnahmen beinhalten, wodurch für jede Person ein Pflegeplan definiert ist. Zum anderen muss das Pflegepersonal manuell bestätigen, welche der geplanten Maßnahmen zu welchem Zeitpunkt durchgeführt wurden. Der von einer in der Kontroll- und Protokollierungseinheit 1 angeordneten Verarbeitungseinheit 5 ermittelte Pflegestatus, der im wesentlichen anhand eines Vergleichs der geplanten mit den durchgeführten Pflege- bzw. Medikationsmaßnahmen erstellt wird, hängt in diesem Fall ausschließlich von den Eingaben des Pflegepersonals sowie von dem aktuellen Zeitpunkt ab.

Wesentlich dabei ist, dass sich der Pflegestatus im Laufe der Zeit ändern kann, was beispielsweise der Fall ist, wenn eine bestimmte Zeitvorgabe für eine geplante Pflegemaßnahme nicht eingehalten wurde, wodurch sich der Pflegestatus für die betroffene Person verschlechtert. Da das erfindungsgemäße System zu jedem Zeitpunkt den aktuellen Pflegestatus anzeigen soll, ist dieses mit einer internen Uhr 6 ausgestattet, so dass die zeitliche Entwicklung des Pflegestatus von der Verarbeitungseinheit 5 überwacht werden kann.

Fig. 1 zeigt bereits eine Weiterbildung des zuvor beschriebenen Basissystems. Hierbei sind die einzelnen Zimmer 10 mit den darin angeordneten Betten 20 über ein Busleitungssystem 7 mit der Kontroll- und Protokollierungseinheit 1 verbunden. Dabei können sich an den Betten 20 mehrere - nicht dargestellte - Überwachungseinrichtungen befinden, welche Körperfunktionen der Patienten überwachen und die ermittelten Messdaten an die Kontroll- und Protokollierungseinheit 1 übermitteln. Bei diesen Überwachungseinrichtungen kann es sich beispielsweise um Vorrichtungen zur Messung der Körpertemperatur, des Puls oder des Blutdrucks der Patienten handeln. Die ermittelten und über das Busleitungssystem 7 übertragenen Messwerte können dann von der Verarbeitungseinheit 5 der Überwachungs- und Protokollierungseinheit 1 zusätzlich zu den in der Datenbank 2 erhaltenen Informationen bei der Ermittlung des Pflegestatus berücksichtigt werden.

Bei diesem erweiterten System kann der Pflegestatus beispielsweise automatisch in eine schlechtere Kategorie wechseln, wenn dies aufgrund der Meßergebnisse der Überwachungseinrichtungen erforderlich erscheint, z.B. wenn die Meßergebnisse anzeigen, dass der Patient eine erhöhte Temperatur aufweist. Auf diese Weise ist für das Pflegepersonal unmittelbar ersichtlich, ob zu den ursprünglich geplanten Maßnahmen möglicherweise eine ergänzende Verabreichung von Medikamenten oder Durchführung zusätzlicher Maßnahmen erforderlich ist. Insbesondere kann die Überwachungs- und Protokollierungseinheit 1 in einem solchen Fall auch von sich aus zusätzliche Pflege- oder Medikationsmaßnahmen vorschlagen (die ggf. von einem Arzt oder dem Pflegepersonal zu bestätigen sind) und damit den Pflegeplan für die betroffene Person aktiv verändern.

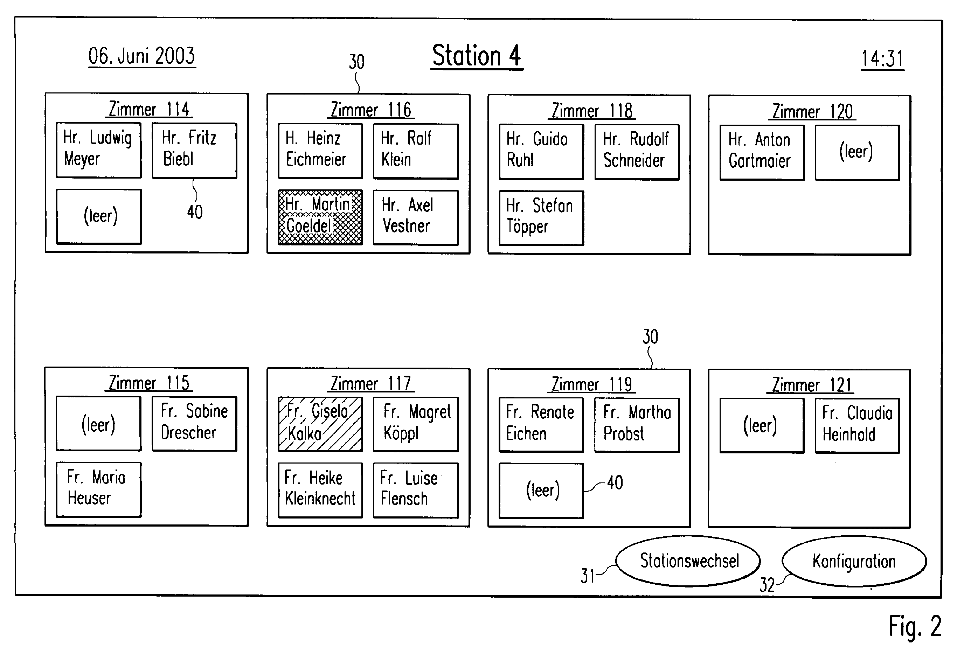

Ein wesentliches Merkmal der vorliegenden Erfindung ist, dass auf der Anzeigeeinheit 3 übersichtlich dargestellt ist, ob die Pflege der Personen entsprechend den Planungen durchgeführt wurde oder nicht. Hierdurch wird dem Pflegepersonal eine einfache und unmittelbare Rückmeldung ermöglicht, welche anzeigt, ob noch bestimmte Tätigkeiten durchzuführen sind. Die Darstellung des Pflegestatus der verschiedenen Personen erfolgt dabei vorzugsweise entsprechend der Übersichtsdarstellung gemäß Fig. 2, die nachfolgend erläutert werden soll.

Fig. 2 zeigt eine auf der Anzeigeeinheit 3 dargestellte Übersichtsanzeige, welche den Pflegestatus der Patienten einer Station darstellt. Um eine besonders einfach zu verstehende Übersicht zu erhalten, sind dabei die verschiedenen Zimmer mit den darin befindlichen Personen grafisch dargestellt, wobei die Darstellung in etwa der räumlichen Anordnung der verschiedenen Zimmer entsprechend sollte. Jedes Zimmer wird dabei symbolisch durch ein großes Rechteck 30 dargestellt, wobei in der Kopfzeile des Rechtecks zunächst die Zimmemummer und anschließend die verschiedenen Betten dargestellt sind. Die als kleinere Rechtecke dargestellten Betten 40 enthalten dabei jeweils den Namen der sich darin befindenden Person, wobei nicht belegte Betten als weißes Rechteck mit der Information "leer" gekennzeichnet sind.

Die Kopfzeile der Übersichtsdarstellung beinhaltet darüber hinaus Informationen hinsichtlich der Nummer der Kranken- oder Pflegestation sowie des Datums und der aktuellen Zeit. Diese Angaben sind insofern von Bedeutung, als der dargestellte Pflegestatus immer von der Kontroll- und Protokollierungseinheit aktualisiert wird, so dass für das Pflegepersonal unmittelbar ersichtlich ist, bei welchen Personen zum aktuellen Zeitpunkt noch Maßnahmen durchgeführt werden müssen.

In der Fußzeile der Darstellung befinden sich zwei Schaltflächen, wobei über die erste Schaltfläche 31 die Station ausgewählt werden kann, zu der ein entsprechender Pflegestatus dargestellt werden soll. Dies eröffnet die Möglichkeit, auf einer zentralen Anzeigeeinheit den Pflegestatus verschiedener Stationen darzustellen, was beispielsweise während der Nachtschicht des Pflegepersonals wünschenswert ist, da hier einzelne Personen des Pflegepersonals auch für mehrere Stationen zuständig sein können. Ferner wird damit die Möglichkeit eröffnet, von einer zentralen Stelle aus die Pflege der Personen zu überwachen. Eine zweite Schaltfläche 32 ermöglicht die Auswahl eines Konfigurations-Menüs, bei dem beispielsweise die Anzahl und Anordnung der verschiedenen Patientenzimmer verändert werden kann. Ferner besteht die Möglichkeit, die Bettenzahl der verschiedenen Zimmer zu verändern und damit neuen Verhältnisses anzupassen.

Wie bereits erwähnt wurde, ist die Anzeigeeinheit 3 vorzugsweise als Touch-Screen ausgestaltet. Die Schaltflächen 31 und 32 können somit unmittelbar durch eine Berührung der Oberfläche des Bildschirmes an der entsprechenden Stelle angewählt werden.

Die symbolisch die einzelnen Betten darstellenden Rechtecke 40 sind ebenfalls als Schaltflächen ausgebildet, so dass bei einer Berührung der entsprechenden Schaltfläche automatisch der dem Bett zugeordnete Pflegeplan - beispielsweise in einem neuen Fenster - angezeigt wird. Anzumerken ist, daß gemäß einer vorteilhaften Variante die Darstellung der einzelnen Betten noch zusätzliche Informationen enthalten kann.

Gemäß der vergrößerten Darstellung in Fig. 2a ist vorgesehen, daß jedes Rechteck-Symbol 40 beispielsweise an seinem unteren Rand noch mit einer zusätzlichen Informationsleiste 41 versehen ist, welche in den entsprechenden Feldern 42-44 Informationen hinsichtlich neuer ärztlicher Anweisungen sowie neuer Indikationen (sofern vorhanden) darstellt. Diese neuen Informationen können ggf. farblich abgehoben in verschiedenen Farben dargestellt werden, so daß der Übersichtsdarstellung neben dem Pflegestatus auch sofort wichtige neue Anweisungen und Informationen bzw. Auffälligkeiten hinsichtlich der zu pflegenden Personen entnommen werden können.

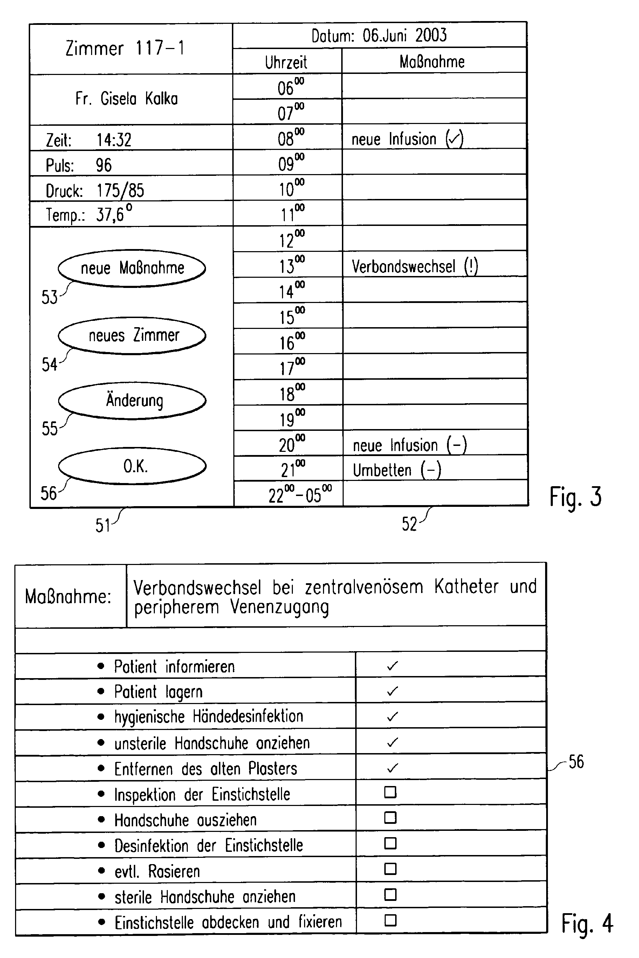

Die sich nunmehr bei einer Berührung des linken oberen Betts bzw. Rechtecks in Zimmer 117 ergebene sog. Kalenderdarstellung ist in Fig. 3 dargestellt. Diese Darstellung beinhaltet in der linken Spalte eine Statusanzeige 51 sowie auf der rechten Seite die Darstellung eines der entsprechenden Person zugeordneten Pflegeplans 52.

Die Statusanzeige 51 der linken Spalte beinhaltet neben den Informationen hinsichtlich der Zimmer- und Bettennummer (im vorliegenden Fall Zimmer 117, Bett Nr. 1) sowie des Namens der zugeordneten Person auch noch Anzeigen über die aktuelle Zeit, den aktuellen Puls, den Blutdruck und die Köpertemperatur. In dem dargestellten Ausführungsbeispiel sind dementsprechend an dem entsprechenden Bett der Patientin Überwachungseinrichtungen angeordnet, welche die Messwerte der entsprechenden Körperfunktionen ermitteln und an die Kontroll- und Protokollierungseinheit übermitteln. Darüber hinaus finden sich im unteren Bereich der Statusanzeige 51 noch vier weitere Schaltflächen 53 bis 56, deren Funktion später erläutert wird.

Der Zeitplan 52 auf der rechten Seite der Kalenderdarstellung beinhaltet Informationen hinsichtlich der zu bestimmten Zeitpunkten durchzuführenden Maßnahmen. Im vorliegenden Fall ist beispielsweise für 8 Uhr das Anlegen einer neuen Infusion geplant, während um 13 Uhr ein Verbandswechsel, um 20 Uhr das nochmalige Anlegen einer Infusion und um 21 Uhr ein Umbetten der Patientin geplant ist. Das Einfügen einer neuen oder zusätzlichen Pflege- oder Medikationsmaßnahme kann durch eine Betätigung der Schaltfläche 53 erfolgen, über die ein - nicht dargestelltes - Untermenü angewählt wird, in dem das beabsichtigte Datum, der Zeitpunkt und die Art der neu geplanten Maßnahme eingegeben werden kann. Mit der weiteren Schaltflächen 54 hingegen kann die Patientin einem neuen Zimmer zugewiesen werden, wobei die in dem Pflegeplan enthaltenen Informationen beibehalten werden. Mit Hilfe der Schaltfläche 55, kann der entsprechenden Zimmer- und Bettnummer ein vollkommen neuer Patient zugewiesen werden, mit Hilfe der Schaltfläche 56 wird wieder in die Übersichtsanzeige gemäß Fig. 3 gewechselt.

Die in dem Kalender bzw. Zeitplan 52 dargestellten Pflegemaßnahmen beinhalten ergänzend die Information, ob diese Maßnahmen bereits durchgeführt wurden. Im dargestellten Beispiel befindet sich z.B. hinter der für 8 Uhr geplanten Pflegemaßnahme ("neue Infusion") ein Häkchen. Dies stellt symbolisch dar, dass von dem Pflegepersonal bestätigt wurde, dass diese Maßnahme tatsächlich durchgeführt wurde. Die Maßnahmen um 20 und 21 Uhr liegen hingegen noch in der Zukunft, weshalb bislang noch keine Bestätigung durch das Pflegepersonal erfolgen konnte, was durch einen einfachen Strich symbolisiert wird.

Der Zeitpunkt für die Maßnahme "Verbandswechsel" (13 Uhr) liegt allerdings bereits in der Vergangenheit, wobei durch das Pflegepersonal noch nicht bestätigt wurde, dass diese Maßnahme auch durchgeführt wurde. In diesem Fall erscheint als Symbol hinter der Pflegemaßnahme ein Ausrufezeichen, welches anzeigt, dass die Maßnahme bereits hätte durchgeführt werden sollen, eine Durchführung allerdings noch nicht bestätigt wurde. Dies bedeutet, dass die für die Patientin vorgesehenen Pflegemaßnahmen noch nicht vollständig in der geplanten Weise durchgeführt wurden, dass also der aktuelle Pflegestatus für diese Person unzureichend ist. Auf der Übersichtsdarstellung in Fig. 2 wird dementsprechend das dieser Person zugeordnete Bett 40 rot hinterlegt dargestellt. Hierdurch ist anhand der Gesamtübersicht für das Pflegepersonal erkenntlich, bei welchen Patienten noch Handlungen vorzunehmen sind.

Der den einzelnen Patienten zugeordnete Pflegestatus ist somit in erster Linie davon abhängig, ob die bis zum aktuellen Zeitpunkt vorgesehenen Pflegemaßnahmen für die verschiedenen Personen vollständig durchgeführt wurden. Bei dem in Fig. 3 dargestellten Ausführungsbeispiele war eine zu einem früheren Zeitpunkt vorgesehene Pflegemaßnahme noch nicht durchgeführt, weshalb dieser Patientin der Pflegestatus rot, d.h. "nicht alle Maßnahmen wurden durchgeführt", zugewiesen wurde. Wurden hingegen sämtliche vorgesehenen Maßnahmen durchgeführt, so wird einem Patienten der Pflegestatus grün zugewiesen, was beispielsweise bei den drei weiteren in Zimmer 117 befindlichen Patientin der Fall ist. Darüber hinaus gibt es noch den Pflegestatus gelb bzw. braun, der später noch erläutert ausführlicher wird und besagt, dass zwar nicht alle vorgesehenen Maßnahmen durchgeführt wurden, wobei dies allerdings aufgrund bestimmter Umstände entschuldigt ist.

Hinsichtlich der in Fig. 3 in der Kalenderdarstellung angezeigten Pflegemaßnahmen ist anzumerken, dass diese selbst nochmals eine Schaltfläche darstellen. Wird beispielsweise die für 13 Uhr geplante Darstellung der Maßnahme "Verbandswechsel" angewählt, so ändert sich die Anzeige auf dem Bildschirm in die in Fig. 4 dargestellte

Anzeige. Ein besonderes Merkmal dieser Anzeige ist, dass die einzelnen Schritte für eine standardisierte Durchführung der Pflegemaßnahme als Liste 56 dargestellt sind. Üblicherweise sind in Krankenhäusern oder Pflegeheimen fast alle Pflegetätigkeiten standardisiert bzw. beschrieben, um eine einheitliche Pflege und Behandlung zu gewährleisten. Diese Standards oder Anweisungen werden dabei in der Regel von jedem Haus selbst erarbeitet und sind von Haus zu Haus verschieden, was beispielsweise an den verschiedenen Aufgaben oder Schwerpunkten eines Hauses liegt.

Diese Pflegestandards oder Pflegeanweisungen werden im Rahmen des erfindungsgemäßen System in besonderer Weise aufgearbeitet.

So muss das Pflegepersonal zur Bestätigung der Durchführung der gesamten Pflegemaßnahme die Durchführung jedes einzelnen Schritts quittieren, was wiederum durch die Darstellung eines Häkchens angezeigt wird. Im dargestellten Beispiel wurden bislang die ersten fünf Schritte der Pflegemaßnahme durchgeführt, während hingegen die letzten sechs Schritte noch ausstehen. Erst nach einer Bestätigung sämtlicher Pflegeschritte wird dies von dem System als vollständige Durchführung der Pflegemaßnahme akzeptiert, wodurch die entsprechende Bestätigungsanzeige in der Kalenderdarstellung 52 in Figur 3 geändert und der Pflegestatus der Patientin neu ermittelt wird.

Darüber hinaus können die einzelnen Schritte der Pflegemaßnahme wiederum als Schaltfläche ausgebildet sein, wobei bei Anwählen oder Berühren der Schaltfläche ergänzende Informationen zu dem zugehörigen Arbeitsschritt zur Verfügung gestellt werden. Diese Informationen können allgemeine Informationen beinhalten wie z.B. Pflegeziele, Hinweise oder allgemeine Erläuterungen zu diesem spez. Standard, sie können sich allerdings auch auf das zum Einsatz kommende Material beziehen oder Anweisungen zur Durchführung dieses Arbeitsschritts beinhalten. Das auf diese Weise zur Verfügung gestellte Informationsmaterial kann dabei in schriftlicher, bildlicher oder sonstiger Form (z.B. als Video) dargestellt werden, so dass das Pflegepersonal in seiner Tätigkeit bei Bedarf möglichst umfangreich darüber, wie die Tätigkeit, warum die Tätigkeit und auf welche Weise sie ausgeführt werden soll, informiert wird.

Ein Vorteil dieser interaktiven Darstellung des Pflegestandards besteht auch darin, dass eine Änderung des Standards sofort übernommen werden und in die tägliche Arbeit einfließen kann. Dies gilt sowohl für die Verwendung neuen Materials als auch für Veränderungen von Arbeitsabläufen. Die geplanten Pflegemaßnahmen können damit jederzeit entsprechenden den neusten Kenntnissen durchgeführt werden. Die umfangreiche und informative Art der Darstellung ist auch insbesondere dann wichtig, wenn die Pflegemaßnahmen von Pflegekräften, die nicht über die notwendige Erfahrung mit bestimmten Tätigkeiten verfügen, bzw. von Hilfskräften, durchgeführt werden sollen, was in Zukunft im Klinikbereich verstärkt der Fall sein wird. Für diese Personen aber auch für die erfahrenen Pflegekräfte besteht hierdurch die Möglichkeit, sich jederzeit zu informieren und beispielsweise Betriebsanleitungen oder Hinweise der Hersteller neuer Materialien zu betrachten.

Durch diese ergänzende Benutzeroberfläche wird somit eine zuverlässige und genaue Durchführung der geplanten Pflegemaßnahmen gewährleistet, so dass ein einheitlicher und optimaler Pflegestandard gewährleistet ist. Die Benutzeroberfläche kann dabei ferner mit einer Sicherheitsfunktion versehen werden, welche eine Bestätigung für die Durchführung eines bestimmten Schritts durch das Pflegepersonal nur dann zulässt, wenn die zuvor vorgesehenen Schritte bereits vollständig erledigt und ebenfalls bestätigt wurden. Hierdurch ist sichergestellt, dass die geplanten Pflegemaßnahmen Schritt für Schritt abgearbeitet werden. Die dargestellte Benutzeroberfläche ist dabei entsprechend der aus der EP 1 079 316 A9 der Anmelderin bekannten Benutzeroberfläche ausgebildet, auf deren Beschreibung zur näheren Erläuterung verwiesen wird.

Von Bedeutung ist hierbei auch, dass die von dem Pflegepersonal durchgeführten Tätigkeiten dokumentiert werden müssen, um den Nachweis zu erbringen, dass die Personen auch ordnungsgemäß gepflegt werden. Die Dokumentation wird durch das erfindungsgemäße System wesentlich erleichtert, da die Durchführung einer Pflegemaßnahme bzw. - im Rahmen der oben beschriebenen Sicherheitsfunktion - der einzelnen Schritte davon automatisch von dem System registriert wird. Die Dokumentation kann dann auf einfache Weise von dem System abgerufen oder ausgedruckt werden. Die standardisierten und/oder von ärztlicher Seite angeordneten Tätigkeiten des Pflegepersonals können ferner mit Zeiten, Kosten und Materialmengen hinterlegt werden, so dass automatisch Daten für eine patientenbezogene Abrechnung zur Verfügung stehen.

Ein Punkt, bei dem die Dokumentation von durchgeführten Maßnahmen eine besonders wichtige Rolle spielt, ist die Verabreichung von Medikamenten. Auch hierbei ist selbstverständlich eine Bestätigung durch das Pflegepersonal erforderlich wobei gemäß einer besonders bevorzugten Variante vorgesehen sein kann, daß das verabreichte Medikament mit Hilfe eines Scanners zuvor identifiziert werden muß. Da Medikamentenverpackungen in der Regel einen das Medikament eindeutig kennzeichnenden Strich- oder Barcode aufweisen, kann durch den Einsatz eines diesen Code lesenden Scanners für das erfindungsgemäße Überwachungssystem erkennbar gemacht werden, welches Medikament das Pflegepersonal oder der Arzt verabreichen möchte. Erst, wenn der Abgleich ergibt, daß das Medikament entsprechend dem Pflegeplan auch tatsächlich verschrieben ist, kann das Überwachungssystem durch eine entsprechende Anzeige die Verabreichung freigeben. Hierdurch wird zum einen eine besonders sicher Überwachung der Medikamentenverabreichung erzielt, zum anderen wird speziell dieser besonders wesentliche Teil der Patientenpflege besonders sorgfältig protokolliert.

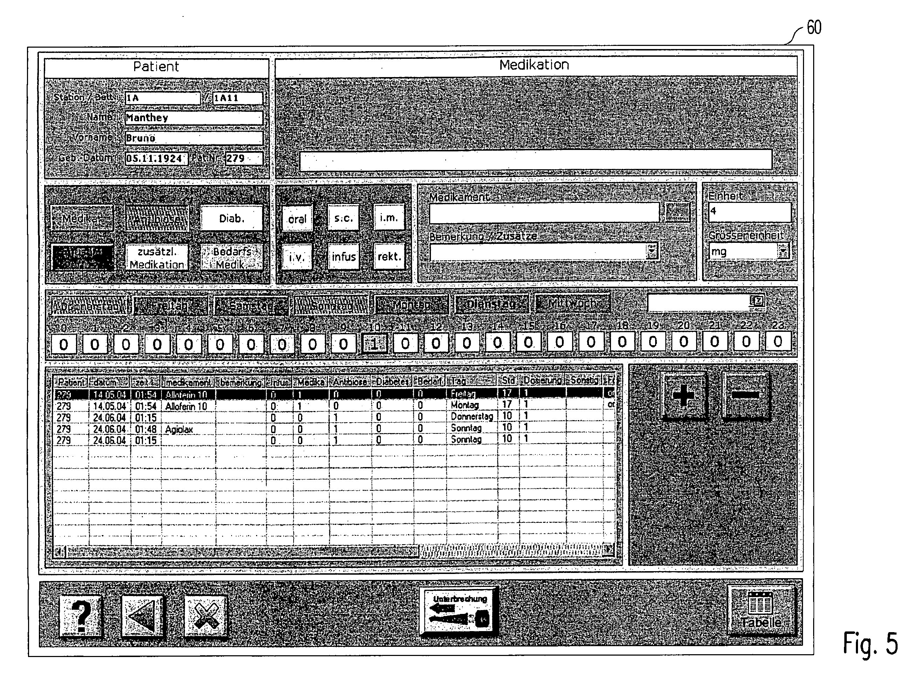

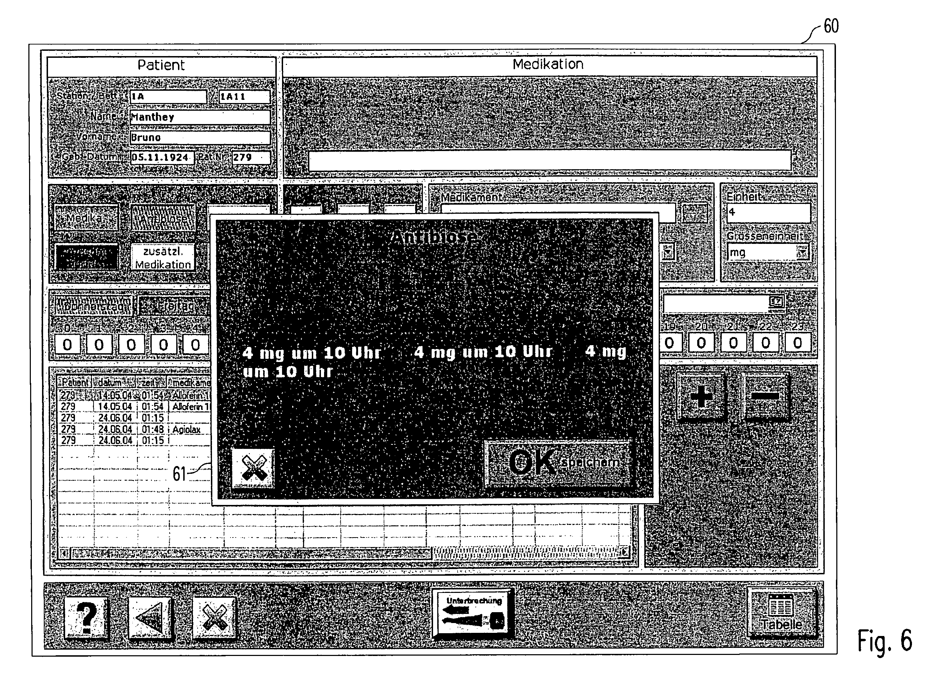

Auch bei der Planung der Medikamentenverabreichung sollte besondere Sorgfalt herrschen, was gemäß einer anderen Weiterbildung der vorliegenden Erfindung durch eine erweiterte Medikations-Anzeige 60 erzielt wird, wie sie in den Figuren 5 und 6 dargestellt ist und die beispielsweise durch eine Betätigung der Schaltfläche 53 in der Anzeige gemäß Fig. 3 aufgerufen werden kann. Dabei ist entsprechend der Darstellung nach Fig. 6 vorgesehen, daß bei der Eingabe einer neuen Information bzw. einer Änderung diese Information nochmals vergrößert in einem eigenen Fenster 61 dargestellt wird und vor einem endgültigen Speichern nochmals explizit bestätigt werden muß. Sinn dieser Maßnahme ist, daß dem Pflegepersonal bzw. dem Arzt nochmals bewusst die von ihm angeordnete Maßnahme vor Augen geführt wird, so daß sich dann später das ausführende Pflegepersonal darauf verlassen kann, daß die von dem erfindungsgemäßen System vorgeschriebenen Maßnahmen und Medikamente auch tatsächlich gewollt waren. Auch in diesem Punkt soll also ein besonders hoher Sicherheitsstandard erzielt werden.

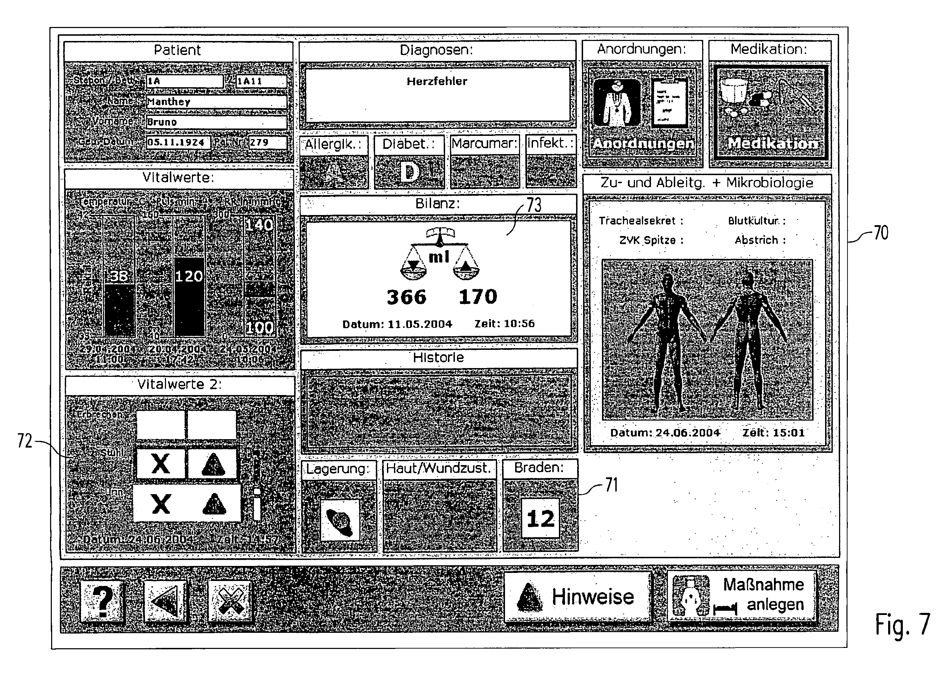

Auch die in der Statusanzeige 51 enthaltenen Anzeigen über die aktuelle Zeit, den aktuellen Puls, den Blutdruck und die Köpertemperatur des Patienten können als Schaltfeld ausgebildet sein, um zu einer erweiterten Patientenzustandsanzeige 70 zu gelangen, die in Fig. 7 dargestellt ist. Diese erweitere Anzeige 70 enthält neben den Darstellungen aller wesentlichen Vitalwerte noch eine Vielzahl weiterer Informationen hinsichtlich das aktuellen Ist-Zustands des Patienten. Eine insbesondere für die Pflege von bettlägerigen Personen wichtige Funktion ist die hierbei realisierte Überwachung des Risikos von Wundliegen (Dekubitus). Hierbei handelt es sich um das Auftreten von Wunden, die darauf zurückzuführen sind, daß nicht ausreichend mobile Personen immer in der gleichen Position liegen.

Das Risiko des Wundliegens wird oftmals mit Hilfe von Skalen bewertet, beispielsweise mit der sog. Braden-Skala. Die erweiterte Patientenzustandsanzeige enthält nunmehr auch eine Anzeige 71 des für den entsprechenden Patienten vorliegenden Skalen-Levels, wobei zum einen der Skalenwert als Zahl dargestellt ist und ferner das sich durch diesen Wert ergebende Risiko durch eine entsprechende farbliche Hinterlegung (z.B. wiederum in den Farben Grün, Gelb und Rot) des Skalenwerts angezeigt wird. Dieser Risikowert kann dabei insbesondere auch bei der Bestimmung des aktuellen Pflegestatus berücksichtigt werden.

Weitere zur Bewertung des aktuellen Zustands wesentliche Informationen, die der erweiterten Patientenzustandsanzeige entnommen werden können, sind sog. Input-Output-Informationen 72, 73 beispielsweise hinsichtlich des Flüssigkeitshaushalts der zu pflegenden Person. Hierbei wird gleichzeitig auch eine Bilanzierung durchgeführt, wobei bei Auffälligkeiten in dieser Bilanzierung wiederum Warninformationen abgegeben und ggf. Anpassungen des Pflegestatus durchgeführt werden.

Anzumerken ist, daß die erweiterte Patientenzustandsanzeige 70 auch unmittelbar über die Übersichtsanzeige von Fig. 2 aufgerufen werden könnte, beispielsweise durch eine besondere Betätigung (z.B. Doppel-Klick) der entsprechenden Schaltflächen.

Wie bereits oben erläutert wurde, gibt es neben dem Pflegestatus grün ("alle Maßnahmen durchgeführt") und rot ("mindestens eine Maßnahme unentschuldigt noch nicht durchgeführt") noch den Pflegestatus gelb bzw. braun. Dieser besagt, dass eine für einen früheren Zeitpunkt geplante Pflegemaßnahme noch nicht durchgeführt wurde, wobei die Verzögerung allerdings entschuldigt ist. Dies kann beispielsweise für den Fall auftreten, dass ein Patient zur Zeit nicht anwesend ist, weil er bereits mit der Durchführung einer anderen Maßnahme beschäftigt ist.

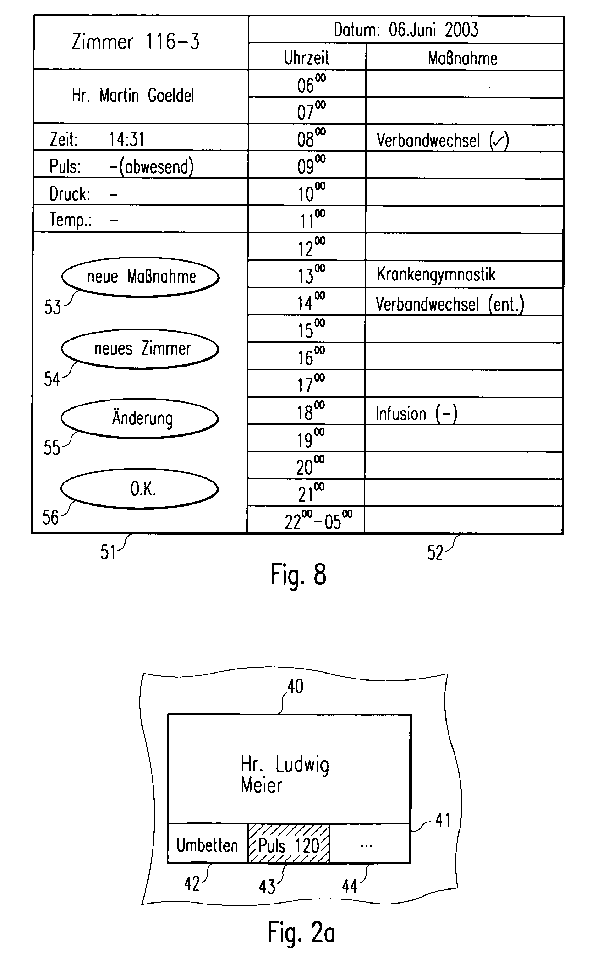

Bei dem in Fig. 8 dargestellten Ausführungsbeispiel war für den Patienten beispielsweise um 13 Uhr ein Termin zur Krankengymnastik vorgesehen, von dem er bis jetzt noch nicht zurückgekehrt ist, weshalb der für 14 Uhr geplante Verbandswechsel noch nicht durchgeführt werden konnte. Die Nichtdurchführung des Verbandwechsels um 14 Uhr ist in diesem Fall entschuldigt, weshalb die Statusanzeige hinter dieser Maßnahme nicht ein Ausrufezeichen beinhaltet, sondern lediglich angibt, dass die Nichtdurchführung der Maßnahme als entschuldigt anzusehen ist. In diesem Fall wechselt der von der Kontroll- und Protokollierungseinheit ermittelte und in der Übersichtsdarstellung von Fig. 2 angezeigte Pflegestatus nicht zu rot, sondern zu gelb bzw. braun, um anzuzeigen, dass bei diesem Patienten ein Maßnahme zu einem späteren Zeitpunkt noch durchgeführt werden sollte.

Der typische Arbeitsablauf einer Pflegekraft unter Zuhilfenahme des erfindungsgemäßen Systems stellt sich dann wie folgt dar:

Die Pflegekraft nimmt ihren - in Fig. 1 dargestellten - Pflegewagen 100 mit dem Touch-Screen 103 in ein bestimmtes Krankenzimmer. Die Darstellung auf dem Monitor entspricht zu diesem Zeitpunkt der Übersichtsdarstellung in Fig. 2. Nun geht die Pflegekraft zu einem ersten Patienten und ruft durch Berührung des entsprechenden Symbols auf dem Touch-Screen 103 die zugehörigen Informationen auf, d.h., die Anzeige wechselt zu der entsprechenden Kalenderdarstellung gemäß Fig. 3, in der die noch auszuführenden Pflegemaßnahmen in zeitlicher Reihenfolge aufgelistet sind.

Tippt die Pflegekraft nun auf die nächste auszuführende Pflegemaßnahme, so erschient die in einzelne Schritte aufgeteilte Pflegeanweisung gemäß der Darstellung in Fig. 4 als interaktive Benutzeroberfläche.

Während der Durchführung der Pflegemaßnahme quittiert die Pflegekraft die Ausführung der einzelnen Schritte der Anweisung indem sie auf das Feld neben der Darstellung des Arbeitsschrittes tippt. Sollte die Pflegekraft während der Arbeit eine Information benötigen, z.B. Hinweise zur Anwendung eines neuen Materials oder zur Ausführung einer bestimmten Verbandstechnik, so kann sie auf die entsprechende Schaltfläche tippen, woraufhin die angeforderten Informationen in schriftlicher oder bildlicher Form oder sogar als Video dargestellt werden.

Für die Pflegekraft besteht ferner die Möglichkeit, sofern dies ein Schritt der Pflegemaßnahme erfordert, erhobene Daten (z.B. Blutdruck, Puls, Temperatur...) über den Touch-Screen 103 einzugeben. Ebenso können Auffälligkeiten, zusätzlich benötigte Zeit oder Materialien für den Patienten festgehalten werden. Mit Bestätigung des letzten Arbeitsschrittes wird dann die Durchführung dieser Pflegemaßnahme insgesamt bestätigt und die Anzeige wechselt wieder zu der Kalenderdarstellung.

Nach Beendigung der Arbeit an dem Patienten tippt die Pflegekraft die Schaltfläche "O.K." an - die Anzeige wechselt daraufhin zur Übersichtsdarstellung - und wechselt zu dem nächsten Patienten.

Sollte die Pflegekraft eine Anweisung vergessen haben, so stellt das System nach Verstreichen der vorgegebenen Zeit in der Übersichtsdarstellung den Patienten in rot dar als Signal für eine nicht zeitgerechte Ausführung einer Anweisung bzw. einen ungenügenden Pflegestatus. Wenn allerdings eine Tätigkeit nicht ausgeführt werden konnte, weil z.B. der Patient nicht im Zimmer ist, so gibt die Pflegekraft dies in das System ein und der Patient wird lediglich in gelb dargestellt als Zeichen für eine begründeter Weise nicht ausgeführte Anweisung.

Nach Anwählen der Schaltfläche "O.K." sind alle eingegebenen Daten in dem System gespeichert und es ist festgehalten, welche Pflegekraft wann, an welchem Patienten, was und wie gemacht hat. Darüber hinaus werden die patientenspezifischen Werte (Blutdruck, Puls, Temperatur...) festgehalten. Das System speichert darüber hinaus auch Informationen hinsichtlich der verbrauchten Materialien, der Dauer und der Kosten der Pflegemaßnahmen, sofern den einzelnen Arbeitsschritten entsprechende Zeiten und Kosten zugeordnet wurden. Hierdurch werden alle zur Dokumentation notwendigen Daten festgehalten, die nach Wunsch und Erfordernis ausgedruckt werden können.

Anzumerken ist, dass die Darstellung der Benutzeroberfläche selbstverständlich auch variiert werden kann. So kann insbesondere in der Kalenderdarstellung auch ein längerer Zeitraum des Pflegeplans, beispielsweise eine Woche dargestellt werden. Ferner könnte bereits die Darstellung gemäß Fig. 4 mit Symbolen oder Icons versehen werden, welche die einzelnen Arbeitsschritte kennzeichnen. Diese Icons könnten dann Schaltflächen bilden, die anzuwählen sind, um nähere Informationen über den Arbeitsschritt zu erhalten.

Insgesamt ergibt sich damit, dass das versehentliche Vergessen einer geplanten Pflegemaßnahme nahezu ausgeschlossen ist, da das System jede noch auszuführende Maßnahme anzeigt und darüber hinaus auch darüber informiert, ob geplante Maßnahmen in Zeit bzw. nicht ausgeführt wurden. Nicht vergessene aber aus anderen Gründen nicht ausgeführte Maßnahmen werden ebenfalls sichtbar gemacht und der Grund für die Nicht-Ausführung festgehalten. Ferner kann das System durch die Überwachungseinheiten für die Patienten ergänzt werden, wodurch die Überwachung von Stationen wesentlich erleichtert wird.

Durch die vorliegende Erfindung wird somit eine Möglichkeit geschaffen, auf einfache Weise die Pflege einer Vielzahl von Personen zu verwalten bzw. zu überwachen und dem Pflegepersonal die Möglichkeit zu geben, schnell festzustellen, inwiefern noch Maßnahmen durchgeführt werden müssen. Hierdurch wird zum einen die Arbeit des Pflegepersonals deutlich vereinfacht, zum ändern wird eine optimale und zuverlässige Pflege der Personen gewährleistet.

## Patentansprüche

1. Interaktives System zur Kontrolle und Protokollierung der Pflege und/oder Medikation mindestens zweier Personen, aufweisend
a) eine Datenbank (2), welche Information bezüglich der Art sowie des Zeitpunkts von an den Personen durchzuführenden Pflege- und/oder Medikationsmaßnahmen beinhaltet,
b) eine Kontroll- und Protokollierungseinheit (1), welche auf Basis der in der Datenbank (2) enthaltenen Information sowie unter Berücksichtigung von Eingaben des Pflegepersonals einen dem aktuellen Zeitpunkt entsprechenden Pflegestatus für die mindestens zwei Personen ermittelt, sowie
c) eine Anzeigeeinheit (3), auf welcher der von der Kontroll- und Protokollierungseinheit (1) ermittelte Pflegestatus dargestellt ist.

2. System nach Anspruch 1,
**dadurch gekennzeichnet,**
**dass** die Kontroll- und Protokollierungseinheit (1) den Pflegestatus anhand eines Vergleichs der geplanten mit den durchgeführten Pflege- und/oder Medikationsmaßnahmen ermittelt.

3. System nach Anspruch 2,
**dadurch gekennzeichnet,**
**dass** der von der Kontroll- und Protokollierungseinheit (1) ermittelte Pflegestatus auf der Anzeigeeinheit (3) symbolisch dargestellt ist.

4. System nach Anspruch 3,
**dadurch gekennzeichnet,**
**dass** die symbolische Darstellung des ermittelten Pflegestatus eine farbliche Kodierung umfaßt.

5. System nach Anspruch 4,
**dadurch gekennzeichnet,**
**dass** die Darstellung des zu einer Person ermittelten Pflegestatus durch eine der Kodierung entsprechende farbliche Hinterlegung des Namens der Person erfolgt.

6. System nach Anspruch einem der Ansprüche 1 bis 5,
**dadurch gekennzeichnet,**
**dass** der von der Kontroll- und Protokollierungseinheit (1) ermittelte Pflegestatus einer der drei Kategorien "Alle Maßnahmen erledigt", "Maßnahme nicht erledigt, aber entschuldigt" sowie "Maßnahme nicht erledigt" entspricht.

7. System nach Anspruch 4 oder 5 sowie Anspruch 6,
**dadurch gekennzeichnet,**
**dass** die drei Kategorien "Alle Maßnahmen erledigt", "Maßnahme nicht erledigt, aber entschuldigt" und "Maßnahme nicht erledigt" durch die Farben Grün, Gelb bzw. Braun und Rot kodiert sind.

8. System nach einem der vorherigen Ansprüche,
**gekennzeichnet durch**
mindestens eine Überwachungseinrichtung, welche eine oder mehrere Körperfunktionen einer zu pflegenden Person überwacht, wobei die Kontroll- und Protokollierungseinheit (1) bei der Ermittlung des Pflegestatus die von der Überwachungseinrichtung erfaßten Meßwerte berücksichtigt.

9. System nach Anspruch 8,
**dadurch gekennzeichnet,**
**dass** es sich bei der von der Überwachungseinrichtung überwachten Körperfunktion um die Körpertemperatur der zu pflegenden Person handelt.

10. System nach Anspruch 8 oder 9,
**dadurch gekennzeichnet,**
**dass** es sich bei der von der Überwachungseinrichtung überwachten Körperfunktion um den Puls der zu pflegenden Person handelt.

11. System nach einem der Ansprüche 8 bis 10,
**dadurch gekennzeichnet,**
**dass** es sich bei der von der Überwachungseinrichtung überwachten Körperfunktion um den Blutdruck der zu pflegenden Person handelt.

12. System nach einem der Ansprüche 8 bis 11,
**dadurch gekennzeichnet,**
**dass** die Kontroll- und Protokollierungseinheit (1) die zu einer Person in der Datenbank (2) enthaltenen Information bezüglich der Art sowie des Zeitpunkts von geplanten Pflege- und/oder Medikationsmaßnahmen unter Berücksichtigung der von den Überwachungseinrichtungen erhaltenen Daten verändert.

13. System nach Anspruch 12,
**dadurch gekennzeichnet,**
**dass** die von der Kontroll- und Protokollierungseinheit (1) vorgenommenen Änderungen der in der Datenbank (2) enthaltenen Information von einer berechtigten Person bestätigt werden müssen.

14. System nach einem der vorhergehenden Ansprüche,
**dadurch gekennzeichnet,**
**dass** die Anzeigeeinheit (3) als Touch-Screen ausgebildet ist.

15. System nach einem der vorhergehenden Ansprüche,
**dadurch gekennzeichnet,**
**dass** die einzelnen Schritte einer durchzuführenden Pflege- und/oder Medikationsmaßnahme als Liste (56) dargestellt sind, wobei zur Bestätigung der Durchführung einer Pflege- und/oder Medikationsmaßnahme jeder einzelne Schritt zu bestätigen ist.

16. System nach Anspruch 15,
**dadurch gekennzeichnet,**
**dass** die Durchführung eines einzelnen Schritts einer Pflege- und/oder Medikationsmaßnahme nur dann bestätigt werden kann, wenn die Durchführung aller zuvor vorgesehen Schritte bestätigt wurde.

17. System nach Anspruch 15 oder 16,
**dadurch gekennzeichnet,**
**dass** die einzelnen Schritte als Schaltflächen ausgestaltet sind, bei deren Anwahl Informationen hinsichtlich der Durchführung des Arbeitsschritts und/oder zu in dem Arbeitsschritt zu verwendenden Materialien dargestellt werden.

18. System nach einem der Ansprüche 15 bis 17,
**dadurch gekennzeichnet,**
**dass** bei der Verabreichung eines Medikaments dieses zuvor gegenüber dem System identifiziert werden muß.

19. System nach Anspruch 18,
**gekennzeichnet durch**
einen Scanner, über den das zu verabreichende Medikament zu identifizieren ist.

20. System nach einem der vorherigen Ansprüche,
**dadurch gekennzeichnet,**
**dass** eine Änderung oder Ergänzung der Informationen bezüglich der Art sowie des Zeitpunkts von an den Personen durchzuführenden Pflege- und/oder Medikationsmaßnahmen gesondert bestätigt werden muß.

21. System nach Anspruch 21,
**dadurch gekennzeichnet,**
**dass** die Bestätigung der Änderung oder Ergänzung der Informationen in einem gesonderten Fenster (61) der Anzeigeeinheit (3) erfolgt.

22. System nach einem der vorherigen Ansprüche,
**dadurch gekennzeichnet,**
**dass** die Kontroll- und Protokollierungseinheit (1) an einem zentralen Ort eines Krankenhauses bzw. einer Kranken- oder Pflegestation angeordnet ist.

23. System nach einem der Ansprüche 1 bis 21,
**dadurch gekennzeichnet,**
**dass** die Kontroll- und Protokollierungseinheit (1) auf einem Pflegewagen angeordnet ist.
